# EUROPEAN PATENT SPECIFICATION

(11) **EP 3 291 812 B1**
(45) Date of publication and mention of the grant of the patent: **01.09.2021**
(21) Application number: 16790066.1
(22) Date of filing: 05.05.2016
(51) Int. Cl.: A61K 31/44, A61P 27/02, A61K 47/10, A61K 9/08

(54) **INJECTABLE DEPOT FORMULATIONS**
INJIZIERBARE DEPOTFORMULIERUNGEN
FORMULATIONS DE DÉPÔT INJECTABLES

(30) Priority: 05.05.2015 US 201562157257 P
(43) Date of publication of application: 14.03.2018
(73) Proprietor: EyePoint Pharmaceuticals US, Inc., Watertown, MA 02472 (US)
(72) Inventor: ASHTON, Paul, Newton, MA 02460 (US)
(74) Representative: FRKelly
(86) International application number: PCT/US2016/030912
(87) International publication number: WO 2016/179357

(56) References cited:
- EP-A1- 2 156 834
- WO-A1-2013/000909
- WO-A1-2013/090666
- WO-A1-2013/090666
- CN-A- 101 890 018
- WILD A.T. ET AL.: "Concurrent versus sequential sorafenib therapy in combination with radiation for hepatocellular carcinoma", PLOS ONE, vol. 8, no. 6, June 2013 (2013-06), pages 1-13, XP002786764,
- MACUGEN DIABETIC RETINOPATHY STUDY GROUP: "A Phase II Randomized Double-Masked Trial of Pegaptanib, an Anti-Vascular Endothelial Growth Factor Aptamer, for Diabetic Macular Edema", OPHTHALMOLOGY, J. B. LIPPINCOTT CO., PHILADELPHIA, PA, US, vol. 112, no. 10, 1 October 2005 (2005-10-01), pages 1747-1757, XP027635130, ISSN: 0161-6420 [retrieved on 2005-10-01]
- MACUGEN DIABETIC RETINOPATHY STUDY GROUP.: 'A phase II randomized double-masked trial of pegaptanib, an anti-vascular endothelial growth factor aptamer, for diabetic macular edema.' OPHTHALMOLOGY vol. 112.10, 31 October 2005, pages 1747 - 1757., XP027635130
- GRAGOUDAS, EVANGELOS S. ET AL.: 'Pegaptanib for neovascular age-related macular degeneration.' NEW ENGLAND JOURNAL OF MEDICINE vol. 351.27, 30 December 2004, pages 2805 - 2816, XP008064513

## Description

### BACKGROUND

Age related macular degeneration ("AMD") is the leading cause of blindness worldwide, and the World Health Organization estimates that about 14 million people are blind or severely impaired because of AMD. AMD causes the progressive loss of central vision attributable to degenerative and neovascular changes in the macula, a specialized area in the center of the retina. In general, macular degeneration can produce a slow or sudden loss of vision.

Two forms of AMD exist: dry AMD and wet AMD. Typically, AMD begins as dry AMD, which is characterized by the formation of drusen, yellow plaque-like deposits in the macula between the retinal pigment epithelium and the underlying choroid. About 15% of dry AMD patients develop wet AMD, which is characterized by the formation of new blood vessels in the choroid (choroidal neovascularization) and vision loss.

Dry macular degeneration is more common than wet AMD, with about 90% of AMD patients being diagnosed with dry AMD. The dry form of AMD may result from the aging and thinning of macular tissues, depositing of pigment in the macula, or a combination of the two processes. The wet form of the disease usually leads to more serious vision loss. With wet AMD, new blood vessels grow beneath the retina and leak blood and fluid. This leakage causes retinal cells to die and creates blind spots in central vision.

While there is no cure for AMD, treatments for wet AMD exist, such as use of anti-neovascular agents and photodynamic therapy (*i.e.,* laser irradiation of the macula). Anti-neovascular agents for the treatment of wet AMD include agents that block the action of vascular endothelial growth factor (VEGF), thereby slowing angiogenesis. No effective treatment exists for dry AMD.

Wild A.T. et al. PLOS One (2013), 8(6), pages 1-13, and WO2013/090666 disclose injectable formulations comprising poorly water-soluble kinase inhibitors dissolved in liquid carriers comprising water-miscible organic solvents.

### SUMMARY

Disclosed are compositions and methods related to the use of kinase inhibitors in treating macular degeneration and/or retinal vein occlusion. In some embodiments, the kinase inhibitor is sorafenib. In some embodiments, the kinase inhibitor is pazopanib.

### DETAILED DESCRIPTION

### Overview

Some aspects of the invention relate to the finding that the equilibrium solubility of many kinase inhibitors in water is similar to a therapeutically effective concentration of the inhibitor. For example, the solubility of many kinase inhibitors in water is about 1 to about 50 µM (about 0.5 µg/mL to about 25 µg/mL for small molecules with a molecular weight of 500 AMU), which often provides a therapeutically effective concentration. Thus, the equilibrium concentration of the kinase inhibitor in the presence of a depot of a solid (amorphous or crystalline) kinase inhibitor in an aqueous environment is sufficient to maintain a therapeutically effective concentration of the inhibitor in a biological compartment, such as the vitreous of the eye. Accordingly, some aspects of the invention relate to injectable compositions comprising a kinase inhibitor dissolved in a water-miscible organic solvent. The injectable compositions may be administered to a subject to produce a precipitate of the kinase inhibitor *in situ,* as water infiltrates the solution, which may subsequently dissolve to provide sustained release of the kinase inhibitor.

### Definitions

As used herein the specification, "a" or "an" may mean one or more. As used herein in the claim(s), when used in conjunction with the word "comprising", the words "a" or "an" may mean one or more than one. As used herein "another" may mean at least a second or more.

The acronym "PDGF" refers to platelet-derived growth factor.

The term "preventing" is art-recognized, and when used in relation to a condition, is well understood in the art, and includes administration of a composition which reduces the frequency of, or delays the onset of, symptoms of a medical condition in a subject relative to a subject which does not receive the composition. Thus, prevention of macular degeneration includes, for example, reducing the number of diagnoses of macular degeneration in a treated population versus an untreated control population, and/or delaying the onset of symptoms of the macular degeneration in a treated population versus an untreated control population. Prevention of dry macular degeneration includes, for example, reducing the number of detectable drusen in a population of subjects receiving a prophylactic treatment relative to an untreated control population, and/or delaying the appearance of detectable drusen in a treated population versus an untreated control population, e.g., by a statistically and/or clinically significant amount. Prevention of vision loss includes, for example, reducing the magnitude of, or alternatively delaying, vision loss experienced by subjects in a treated population versus an untreated control population.

The term "prophylactic or therapeutic" treatment is art-recognized and includes administration to the host of one or more of the subject compositions. If it is administered prior to clinical manifestation of the unwanted condition (*e.g*., disease or other unwanted state of the subject) then the treatment is prophylactic (*i.e*., it protects the subject against developing the unwanted condition), whereas if it is administered after manifestation of the unwanted condition, the treatment is therapeutic (*i.e.,* it is intended to diminish, ameliorate, or stabilize the existing unwanted condition or side effects thereof).

A "therapeutically effective amount" of a compound refers to a concentration of a kinase inhibitor, which alleviates a symptom, ameliorates a condition, or slows the onset of disease conditions according to clinically acceptable standards for the disorder or condition to be treated, *e.g.,* at a reasonable benefit/risk ratio applicable to any medical treatment.

As used herein, the term "treating" or "treatment" includes reversing, reducing, or arresting the symptoms, clinical signs, and underlying pathology of a condition in a manner to improve or stabilize a subject's condition, or to reduce the severity of disease progression.

The acronym "VEGF" refers to vascular endothelial growth factor.

### A. Injectable compositions comprising kinase inhibitors

Small molecule kinase inhibitors include afatinib, alectinib, apatinib, ASP-3026, axitinib, bafetinib, baricitinib, binimetinib, bosutinib, brigatinib, cabozantinib, canertinib, cediranib, CEP-37440, ceritinib, cobimetinib, copanlisib, crenolanib, crizotinib, CYT387, dabrafenib, damnacanthal, dasatinib, doramapimod, enterctinib, erlotinib, everolimus, filgotinib, foretinib, fostamatinib, gefitinib, grandinin, ibrutinib, icotinib, idelalisib, imatinib, IPI-145, JSI-124, lapatinib, lenvatinib, lestaurtinib, linifanib, masitinib, motesanib, mubritinib, neratinib, nilotinib, nintedanib, pacritinib, palbociclib, pazopanib, pegaptanib, perifosine, PF-06463922, ponatinib, PX-866, quizartinib, radotinib, regorafenib, ruxolitinib, selumetinib, sirolimus, sorafenib, staurosporine, sunitinib, SU6656, temsirolimus, TG101348, tivozanib, toceranib, tofacitinib, trametinib, TSR-011, vandetanib, vemurafenib, and X-396. Large molecule kinase inhibitors include aflibercept, bevacizumab, catumaxomab, panitumumab, ranibizumab, and trastuzumab.

In preferred embodiments, the kinase inhibitor is a tyrosine kinase inhibitor, such as afatinib, alectinib, apatinib, axitinib, bafetinib, baricitinib, binimetinib, bosutinib, brigatinib, cabozantinib, canertinib, cediranib, CEP-37440, ceritinib, cobimetinib, crenolanib, crizotinib, CYT387, damnacanthal, dasatinib, doramapimod, entrectinib, erlotinib, filgotinib, foretinib, fostamatinib, grandinin, gefitinib, ibrutinib, icotinib, imatinib, JSI-124, lapatinib, lestaurtinib, lenvatinib, linifanib, masitinib, motesanib, mubritinib, neratinib, nilotinib, nintedanib, pacritinib, pazopanib, pegaptanib, PF-06463922, ponatinib, quizartinib, radotinib, regorafenib, ruxolitinib, selumetinib, semaxanib, sorafenib, staurosporine, sunitinib, SU6656, TG101348, tivozanib, toceranib, tofacitinib, trametinib, TSR-011, vandetanib, vatalanib, vemurafenib, or X-396. In certain preferred embodiments, the kinase inhibitor is a receptor tyrosine kinase inhibitor. In some embodiments, the kinase inhibitor is not sunitinib.

In some embodiments, the kinase inhibitor is a multi-targeted kinase inhibitor, such as a multi-targeted receptor tyrosine kinase inhibitor.

In certain preferred embodiments, the kinase inhibitor is a VEGF receptor kinase inhibitor, PDGF receptor kinase inhibitor, and/or inflammasome inhibitor. In certain preferred embodiments, the kinase inhibitor is apatinib, axitinib, cabozantinib, cediranib, crenolanib, foretinib, lenvatinib, linifanib, masitinib, motesanib, nintedanib, pazopanib, pegaptanib, regorafenib, semaxanib, sorafenib, sunitinib, tivozanib, toceranib, vandetanib, or vatalanib. In one preferred embodiment, the kinase inhibitor is sorafenib. In another preferred embodiment, the kinase inhibitor is pazopanib.

In certain preferred embodiments, the kinase inhibitor is a BCR/Abl, Src, c-Kit, and/or ephrin receptor inhibitor. In certain preferred embodiments, the kinase inhibitor is bafetinib, bosutinib, dasatinib, imatinib, nilotinib, ponatinib, radotinib, or SU6656.

In certain preferred embodiments, the kinase inhibitor inhibits VEGF receptor kinase activity, *e.g.,* by either binding to a VEGF protein or by binding to a VEGF receptor, thereby inhibiting VEGF receptor kinase activity. In some embodiments, the kinase inhibitor binds to VEGF-A, VEGF-B, VEGF-C, VEGF-D, and/or VEGF-E, *e.g*., thereby inhibiting VEGF receptor kinase activity. In certain embodiments, the kinase inhibitor specifically binds to and inhibits a VEGF receptor, such as VEGFR-1, VEGFR-2, and/or VEGFR-3. In certain preferred embodiments, the kinase inhibitor inhibits angiogenesis.

In certain preferred embodiments, the kinase inhibitor inhibits PDGF receptor kinase activity, *e.g.,* by either binding to PDGF or by binding to a PDGF receptor, thereby inhibiting PDGF receptor kinase activity. In some embodiments, the kinase inhibitor binds to PDGF-A, PDGF-B, PDGF-B, PDGF-C, and/or a homodimer or heterodimer thereof, *e.g*., thereby inhibiting PDGF receptor kinase activity. In certain embodiments, the kinase inhibitor specifically binds to and inhibits a PDGF receptor, such as PDGFR-α and/or PDGFR-β. In certain preferred embodiments, the kinase inhibitor inhibits angiogenesis.

In certain preferred embodiments, the kinase inhibitor inhibits inflammasome activation.

In certain embodiments, the kinase inhibitor inhibits BCR/Abl, Src, c-Kit, and/or an ephrin receptor.

In certain preferred embodiments, the kinase inhibitor is a small molecule. For example, in certain preferred embodiments, the kinase inhibitor has a molecular weight of less than 1000 AMU, such as less than 600 AMU. The kinase inhibitor may have a molecular weight of less than 500 AMU. In some embodiments, the kinase inhibitor has a molecular weight between 300 AMU and 1000 AMU, such as between 300 AMU and 700 AMU, or between 300 AMU and 600 AMU

Because limited solubility in water is a key factor in achieving extended release and avoiding possible toxic side effects, the kinase inhibitor will typically be employed in a form with low solubility, such as a free base or free acid, or a salt with a hydrophobic counterion (such as p-toluenesulfonate).

In some embodiments, the solubility of the kinase inhibitor in water (e.g., at about 37°C) is less than 100 µg/mL, such as less than 10 µg/mL, or even less than 1 µg/mL. In some embodiments, the solubility of the kinase inhibitor in water (e.g., at about 37°C) is between 0.01 µg/mL and 100 µg/mL. In preferred embodiments, the solubility of the kinase inhibitor in water *(e.g.,* at about 37°C) is between 0.1 µg/mL and 10 µg/mL.

In some embodiments, the solubility of the kinase inhibitor in the vitreous humour of a subject (*e.g.,* at about 37°C) is less than 100 µg/mL, such as less than 10 µg/mL, or even less than 1 µg/mL. In some embodiments, the solubility of the kinase inhibitor in the vitreous humour (*e.g*., at about 37°C) is between 0.01 µg/mL and 100 µg/mL. In preferred embodiments, the solubility of the kinase inhibitor in the vitreous humour (*e.g*., at about 37°C) is between 0.1 µg/mL and 10 µg/mL.

In some embodiments, the solubility of the kinase inhibitor in the organic solvent, such as a water-miscible organic solvent, (*e.g*., at about 4°C, 20°C, or 37°C) is greater than 10 µg/mL, such as greater than 100 µg/mL, greater than 1 mg/mL, greater than 10 mg/mL, or even greater than 100 mg/mL. In some embodiments, the solubility of the kinase inhibitor in the organic solvent is between 10 µg/mL and 100 mg/mL, such as between 100 µg/mL and 100 mg/mL, or between 1 mg/mL and 100 mg/mL. The organic solvent may comprise acetic acid, acetone, anisole, 1-butanol, 2-butanol, butyl acetate, dimethyl sulfoxide, ethanol, ethyl acetate, ethyl ether, ethyl formate, formic acid, heptane, isobutyl acetate, isopropyl acetate, methyl acetate, 3-methyl-1-butanol, methylethyl ketone, methylisobutyl ketone, 2-methyl-1-propanol, propylene glycol, pentane, 1-pentanol, 1-propanol, 2-propanol, propyl acetate, and/or tert-butylmethyl ether.

In preferred embodiments, the solvent is ethanol. For example, the solubility of sorafenib in ethanol is about 3.3 mg/mL. Similarly, preferred kinase inhibitors, such as apatinib, axitinib, cabozantinib, cediranib, crenolanib, foretinib, lenvatinib, linifanib, masitinib, motesanib, nintedanib, pazopanib, pegaptanib, regorafenib, semaxanib, sorafenib, sunitinib, tivozanib, toceranib, vandetanib, vatalanib, bafetinib, bosutinib, dasatinib, imatinib, nilotinib, ponatinib, radotinib, and SU6656, are generally soluble in ethanol, *e.g.,* at 1.0 mg/mL or higher.

A human eye comprises about 4 mL of vitreous humour, and typically, at least 50 µL of a composition may be injected into the vitreous without a detrimental increase in intraocular pressure. Accordingly, the solubility of the kinase inhibitor in the organic solvent is preferably at least 80 times greater than the solubility of the kinase inhibitor in water (4 mL / 50 µL = 80). The solubility of the kinase inhibitor in the organic solvent may be at least 100 times greater than the solubility of the kinase inhibitor in water, such as at least 150 times greater, at least 200 times greater, or at least 250 times greater. In more preferable embodiments, the solubility of the kinase inhibitor in the organic solvent is at least about 300 times greater than the solubility of the kinase inhibitor in water, such as at least 350 times greater, at least 400 times greater, at least 450 times greater, at least 500 times greater, at least 600 times greater, at least 700 times greater, at least 800 times greater, at least 900 times greater, or even at least 1000 times greater. The solubility of the kinase inhibitor in the organic solvent may be at least 10³, 10⁴, 10⁵, or 10⁶ greater than the solubility of the kinase inhibitor in water, *e.g.*, for kinase inhibitors with very high solubilities in particular organic solvents.

In some aspects, the invention relates to an injectable composition, comprising a kinase inhibitor (*e.g.,* a poorly water-soluble kinase inhibitor) dissolved in an organic solvent (*e.g.,* a water-miscible organic solvent), wherein the concentration of the kinase inhibitor in the injectable composition is at least 10 times greater than the solubility of the kinase inhibitor in water. In some embodiments, the concentration of the kinase inhibitor in the injectable composition is at least 10, 20, 30, 40, 50, 60, 70, 80, or 90 times greater than the solubility of the kinase inhibitor in water (*e.g*., at 37°C). In preferred embodiments, the concentration of the kinase inhibitor in the injectable composition is at least 100, 150, 200, 250, 300, 350, 400, or 450 times greater than the solubility of the kinase inhibitor in water (*e.g*., at 37°C). In some embodiments, the concentration of the kinase inhibitor in the injectable composition is at least 500, 600, 700, 800, 900, 1000, 10⁴, 10⁵, or 10⁶ times greater than the solubility of the kinase inhibitor in water (*e.g*., at 37°C).

In some aspects, the invention relates to an injectable composition, comprising a kinase inhibitor *(e.g.,* a poorly water-soluble kinase inhibitor) dissolved in an organic solvent *(e.g.,* a water-miscible organic solvent), wherein the concentration of the kinase inhibitor in the injectable composition is at least 10 times greater than the solubility of the kinase inhibitor in the vitreous humour of an eye. In some embodiments, the concentration of the kinase inhibitor in the injectable composition is at least 10, 20, 30, 40, 50, 60, 70, 80, or 90 times greater than the solubility of the kinase inhibitor in the vitreous humour of an eye *(e.g.,* at 37°C). In preferred embodiments, the concentration of the kinase inhibitor in the injectable composition is at least 100, 150, 200, 250, 300, 350, 400, or 450 times greater than the solubility of the kinase inhibitor in the vitreous humour of an eye (*e.g.,* at 37°C). In some embodiments, the concentration of the kinase inhibitor in the injectable composition is at least 500, 600, 700, 800, 900, 1000, 10⁴, 10⁵, or 10⁶ times greater than the solubility of the kinase inhibitor in the vitreous humour of an eye *(e.g.,* at 37°C).

In preferred embodiments, the injectable composition is a liquid solution.

The concentration of the kinase inhibitor in the injectable composition may be at least 100 µg/mL, such as at least 200 µg/mL, at least 300 µg/mL, at least 400 µg/mL, at least 500 µg/mL, at least 600 µg/mL, at least 700 µg/mL, at least 800 µg/mL, at least 900 µg/mL, or at least 1000 µg/mL. In preferred embodiments, the concentration of the kinase inhibitor in the injectable composition is at least 1.0 mg/mL, such as at least 1.1 mg/mL, at least 1.2 mg/mL, at least 1.3 mg/mL, at least 1.4 mg/mL, at least 1.5 mg/mL, at least 1.6 mg/mL, at least 1.7 mg/mL, at least 1.8 mg/mL, at least 1.9 mg/mL, at least 2.0 mg/mL, at least 2.2 mg/mL, at least 2.4 mg/mL, at least 2.5 mg/mL, at least 2.6 mg/mL, at least 2.8 mg/mL, at least 3.0 mg/mL, at least 4.0 mg/mL, at least 5.0 mg/mL, at least 6.0 mg/mL, at least 7.0 mg/mL, at least 8.0 mg/mL, at least 9.0 mg/mL, or at least 10.0 mg/mL. The concentration of the kinase inhibitor in the injectable composition may be at least 20 mg/mL, at least 30 mg/mL, at least 40 mg/mL, at least 50 mg/mL, at least 60 mg/mL, at least 70 mg/mL, at least 80 mg/mL, at least 90 mg/mL, at least 100 mg/mL, at least 200 mg/mL, at least 300 mg/mL, at least 400 mg/mL, at least 500 mg/mL, at least 600 mg/mL, at least 700 mg/mL, at least 800 mg/mL, at least 900 mg/mL, or at least 1000 mg/mL.

The concentration of the kinase inhibitor in the injectable composition may be about 100 µg/mL to about 1 g/ml, such as about 500 µg/mL to about 100 mg/mL, about 700 µg/mL to about 20 mg/mL, or about 900 µg/mL to about 10 mg/mL.

### B. Vials, syringes, and kits comprising injectable compositions

### 1. Vials

In some aspects, the invention relates to a vial comprising an injectable composition, as described herein. For example, the vial may be a sealed, single-use vial. Generally, the vial may comprise any material that is compatible with the organic solvent and the kinase inhibitor, e.g., such that the organic solvent does not dissolve the vial or leach molecules out of the vial and the kinase inhibitor does not leach into the vial. In preferred embodiments, the vial comprises glass.

In preferred embodiments, the vial is sealed. The vial may be sealed with a stopper. In preferred embodiments, the stopper is compatible with the organic solvent. The stopper may comprise, for example, rubber, metal, and/or plastic, such as polytetrafluoroethylene.

In preferred embodiments, the vial is sterile.

In preferred embodiments, the vial comprises at least enough of the injectable composition so that, upon injecting the composition into an eye, at least some of the kinase inhibitor precipitates. For example, the vial may comprise at least 5 µL of the injectable composition, such as at least 10 µL, at least 20 µL, at least 30 µL, at least 40 µL, at least 50 µL, at least 60 µL, at least 70 µL, at least 80 µL, at least 90 µL, or at least 100 µL of the injectable composition. The vial may comprise an excess amount of the injectable composition. For example, the vial may comprise about 5 µL to about 10 mL of the injectable composition, such as about 10 µL to about 5 mL, about 40 µL to about 2 mL, about 50 µL to about 1 mL, or about 60 µL to about 500 µL. The vial may comprise, for example, about 60 µL, 70 µL, 80 µL, 90 µL, 100 µL, 110 µL, 120 µL, 130 µL, 140 µL, 150 µL, 160 µL, 170 µL, 180 µL, 190 µL, 200 µL, 210 µL, 220 µL, 230 µL, 240 µL, 250 µL, 260 µL, 270 µL, 280 µL, 290 µL, 300 µL, 310 µL, 320 µL, 330 µL, 340 µL, 350 µL, 360 µL, 370 µL, 380 µL, 390 µL, or 400 µL of the injectable composition.

The vial may comprise at least 5 µg of the kinase inhibitor, such as at least 10 µg, at least 20 µg, at least 30 µg, at least 40 µg, at least 50 µg, at least 60 µg, at least 70 µg, at least 80 µg, at least 90 µg, or at least 100 µg of the kinase inhibitor. The vial may comprise an excess amount of the kinase inhibitor. For example, the vial may comprise about 5 µg to about 10 mg of the kinase inhibitor, such as about 10 µg to about 5 mg, about 40 µg to about 2 mg, about 50 µg to about 1 mg, or about 60 µg to about 500 µg. The vial may comprise, for example, about 60 µg, 70 µg, 80 µg, 90 µg, 100 µg, 110 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, 300 µg, 310 µg, 320 µg, 330 µg, 340 µg, 350 µg, 360 µg, 370 µg, 380 µg, 390 µg, or 400 µg of the kinase inhibitor.

The size of the vial is not particularly limiting. For example, the vial may have a volume of about 0.1 mL, 0.2 mL, 0.5 mL, 1 mL, 2 mL, 3 mL, 4 mL, 5 mL, 6 mL, 7 mL, 8 mL, 9 mL, or 10 mL. In preferred embodiments, the volume of the vial is about 0.1 mL to about 5 mL, such as 0.1 mL, 0.2 mL, 0.5 mL, 1 mL, 2 mL, or 3 mL.

### 2. Syringes

In some aspects, the invention relates to a syringe, e.g., a syringe comprising an injectable composition as described herein. In preferred embodiments, the syringe is sterile.

In some embodiments, the syringe comprises a Luer taper. The Luer taper may be a slip tip connector or a Luer lock connector. In preferred embodiments, the syringe comprises a male Luer taper.

The size of the syringe is not particularly limiting. Nevertheless, for embodiments in which an injectable composition would be administered to the eye of a subject (e.g., a human subject), a syringe is typically sized to deliver from about 5 µL to about 200 µL of the composition. For example, the syringe may have a volume of about 10 µL, 25 µL, 50 µL, 100 µL, 200 µL, 250 µL, 300 µL, 500 µL, 1 mL, or 1.2 mL *(e.g.,* the syringe might comprise a maximum marked volume that corresponds to any one of the preceding volumes or an increment between any one of the preceding volumes). The syringe may have a volume of about 10 µL to about 10 mL. In preferred embodiments, the syringe has a volume of about 50 µL to about 2 mL, such as about 50 µL, 100 µL, 200 µL, 250 µL, 300 µL, 500 µL, or 1 mL. The syringe may comprise graduated markings, e.g., that correspond to at least one 50-microliter volume.

The syringe may comprise any material that is compatible with the organic solvent and kinase inhibitor, e.g., such that the organic solvent does not dissolve the syringe or leach molecules out of the syringe and the kinase inhibitor does not leach into the syringe. The syringe may comprise, for example, plastic or glass. The syringe may comprise polypropylene or polytetrafluoroethylene. The syringe plunger may comprise rubber or plastic, such as polytetrafluoroethylene.

In some embodiments, the syringe comprises a filter. The filter may comprise polytetrafluorethylene, polyvinylidene fluoride, polyethersulfone, cellulose nitrate, cellulose acetate, regenerated cellulose, cellulose mixed ester, polypropylene, polyamide nylon, polycarbonate, or polyester. In preferred embodiments, the filter is compatible with the organic solvent, e.g., the organic solvent cannot solubilize the filter or significantly alter the pore size of the filter. For example, polytetrafluorethylene, polyvinylidenefluoride, polyethersulfone, cellulose acetate, regenerated cellulose, polypropylene, polyamide nylon, polycarbonate, and polyester filters are compatible with ethanol. The pore size of the filter may be, for example, about 0.2 µm to about 5 µm. The pore size may be 0.2 µm, 0.22 µm, 0.45 µm, 0.5 µm, 0.8 µm, 1.0 µm, 1.1 µm, 1.2 µm, or 5 µm. The filter may be, for example, a 5-micron filter.

In preferred embodiments, the filter is compatible with a Luer tapered syringe. The filter may comprise a slip tip connector or a Luer lock connector. In preferred embodiments, the filter comprises a female Luer taper.

The filter may be a filter needle. In some embodiments, the filter is a 19-gauge filter needle. In some embodiments, the filter is a 1.5 inch long filter needle.

In some embodiments, the syringe comprises a needle. In preferred embodiments, the needle is compatible with a Luer tapered syringe. The needle may comprise a slip tip connector or a Luer lock connector. In preferred embodiments, the needle comprises a female Luer taper.

The needle may comprise metal, such as stainless steel. The needle may be 24 gauge to 32 gauge, or smaller. For example, the needle may be 24, 25, 26, 27, 28, 29, 30, 31, 32, 33, or 34 gauge. The needle may be between about 26 gauge and about 34 gauge, such as between about 27 gauge and about 33 gauge, or between about 28 gauge and about 32 gauge. In preferred embodiments, the needle is 29 gauge, 30 gauge, 31 gauge, or 32 gauge. In preferred embodiments, the needle has a beveled tip.

In some embodiments, the needle has a non-coring tip. A typical problem when inserting a needle with a lumen into any tissue is the phenomena of "coring" of the tissue, where the insertion actually cuts a cylindrical section of tissue that enters the lumen. Such coring, when it occurs in the eye, can exacerbate leakage of eye fluid through the injection site. An alternative is to use a non-coring needle such as a Tuohy needle, which has a curved tip, or a Huber needle, which has a slanted tip. The needle may comprise a point or a blunt tip. Other methods known in the art for avoiding coring may be used such as deflection of the tip of the needle and sharpening portions of the needle point. Any of these tips may be used interchangeably with the syringes disclosed herein, in combination with any other features of the invention.

The length of the needle is not particularly limiting so long as the needle is long enough to pierce an eye *(e.g.,* the sclera of an eye) to inject a composition into the vitreous humour. In some embodiments, the length of the needle is shorter than the diameter of an eye, *e.g.,* to prevent the needle from piercing the back of the eye. The diameter of a human eye, for example, is about 24 mm (*i.e*., about 1 inch). In some embodiments, the length of the needle is between about 5 mm and about 30 mm, such as between about 10 mm and about 20 mm. The length of the needle may be, for example, about 5, 6, 7, 8, 9, 10, 11, 12, 13, 14, 15, 16, 17, 18, 19, 20, 21, 22, 23, 24, 25, 26, 27, 28, 29, or 30 mm. The length of the needle may be about 12.7 mm. In some embodiments, the length of the needle is between about 0.125 inches and about 1 inch, such as between about 0.25 inches and about 0.75 inches. The length of the needle may be about 1/8", 5/32", 3/16", 1/4", 5/16", 1/3", 1/2", 5/8", 3/4", or 1" long. In preferred embodiments, the length of the needle is about 0.5 inches (1/2").

In some embodiments, the syringe comprises a shield for the needle.

In preferred embodiments, the syringe comprises at least enough of the injectable composition so that, upon injecting the composition into an eye, at least some of the kinase inhibitor precipitates. For example, the syringe may comprise at least 5 µL of the injectable composition, such as at least 10 µL, at least 20 µL, at least 30 µL, at least 40 µL, at least 50 µL, at least 60 µL, at least 70 µL, at least 80 µL, at least 90 µL, or at least 100 µL of the injectable composition. The syringe may comprise an excess amount of the injectable composition. For example, the syringe may comprise about 5 µL to about 10 mL of the injectable composition, such as about 10 µL to about 5 mL, about 40 µL to about 2 mL, about 50 µL to about 1 mL, or about 60 µL to about 500 µL. The syringe may comprise, for example, about 60 µL, 70 µL, 80 µL, 90 µL, 100 µL, 110 µL, 120 µL, 130 µL, 140 µL, 150 µL, 160 µL, 170 µL, 180 µL, 190 µL, 200 µL, 210 µL, 220 µL, 230 µL, 240 µL, 250 µL, 260 µL, 270 µL, 280 µL, 290 µL, 300 µL, 310 µL, 320 µL, 330 µL, 340 µL, 350 µL, 360 µL, 370 µL, 380 µL, 390 µL, or 400 µL of the injectable composition.

The syringe may comprise at least 5 µg of the kinase inhibitor, such as at least 10 µg, at least 20 µg, at least 30 µg, at least 40 µg, at least 50 µg, at least 60 µg, at least 70 µg, at least 80 µg, at least 90 µg, or at least 100 µg of the kinase inhibitor. The syringe may comprise an excess amount of the kinase inhibitor. For example, the syringe may comprise about 5 µg to about 10 mg of the kinase inhibitor, such as about 10 µg to about 5 mg, about 40 µg to about 2 mg, about 50 µg to about 1 mg, or about 60 µg to about 500 µg. The syringe may comprise, for example, about 60 µg, 70 µg, 80 µg, 90 µg, 100 µg, 110 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, 300 µg, 310 µg, 320 µg, 330 µg, 340 µg, 350 µg, 360 µg, 370 µg, 380 µg, 390 µg, or 400 µg of the kinase inhibitor.

### 3. Kits

In certain aspects, the invention relates to a kit comprising an injectable composition and a syringe, *i.e.,* as described herein. The kit may comprise a vial (*i.e.,* a vial comprising the injectable composition). For embodiments in which the kit comprises a vial, the syringe typically does not comprise the injectable composition, *i.e.,* the injectable composition is loaded into the syringe from the vial. The kit may further comprise a filter as described herein. The kit may further comprise a needle as described herein, and the kit may optionally comprise a shield for the needle. The kit may further comprise instructions, e.g., a product insert for filtering and/or administering the composition. The kit may further comprise an alcohol wipe or pad, *e.g.,* for cleaning the vial.

### C. Methods for administering an injectable composition

In some aspects, the invention relates to a method for treating or preventing an eye condition or eye disease in a subject, comprising inserting an injectable composition, as described herein, into an eye of the subject. In preferred embodiments, inserting comprises injecting the composition. In preferred embodiments, the composition is inserted (e.g., injected) into the vitreous of the eye. In preferred embodiments, the administration of a composition to the vitreous of an eye, and the subsequent precipitation of a kinase inhibitor, allows for the maintenance of a relatively constant concentration of the kinase inhibitor in the vitreous humour over a period of time.

The method may further comprise filtering the injectable composition prior to administering the injectable composition. The composition may be filtered, for example, using any one of the filters described herein.

In certain embodiments, the compositions are administered to prevent or treat macular degeneration in a subject, e.g., age-related macular degeneration ("AMD"), such as dry AMD and wet AMD. The composition may be administered to prevent the death of retinal pigment epithelial cells. The composition may be administered to prevent *Alu*-RNA induced cytotoxicity. The composition may be administered to inhibit P2X7 activation. The composition may be administered to inhibit caspase-1 activation. The composition may be administered to inhibit angiogenesis. In some embodiments, the compositions are administered to prevent or treat vision loss in a subject, such as vision loss associated with macular degeneration. The composition may be administered to prevent geographic atrophy in an eye. The composition may be administered to prevent or delay the progression of dry AMD to wet AMD

In some embodiments, the compositions are administered to prevent or treat retinal vein occlusion in a subject, e.g., central retinal vein occlusion ("CRVO") or branch retinal vein occlusion ("BRVO"). The compositions may be administered to prevent or treat non-ischemic retinal vein occlusion or ischemic retinal vein occlusion.

In some embodiments, the kinase inhibitor forms a precipitate *(e.g.,* in the vitreous humour of an eye), and the precipitate maintains a therapeutically effective concentration of the kinase inhibitor in the eye for at least 1, 2, 3, 4, 5, 6, 7, or 8 weeks. The precipitate may maintain a therapeutically effective concentration of the kinase inhibitor in the eye for at least 1, 2, 3, 4, 5, or 6 months. In certain embodiments, the precipitate maintains a therapeutically effective concentration of the kinase inhibitor at the site for about 1, 2, 3, 4, 5, 6, 7, or 8 weeks or about 1, 2, 3, 4, 5, 6, 7, 8, 9, 10, or 12 months. In certain preferred embodiments, the composition is configured to maintain a therapeutically effective concentration of the kinase inhibitor at the site for a period of time between about 1 week and about 12 months, such as between about 2 weeks and about 6 months, or between about 4 weeks and about 4 months. Because the rate of delivery is limited by the solubility of the kinase inhibitor in the surrounding fluid, the length of delivery is proportional to the amount of kinase inhibitor administered. Therefore, increasing the dose of the composition will increase the period of time during which an effective concentration of the kinase inhibitor is delivered to the eye.

In some embodiments, the method comprises readministering an injectable composition to the subject (e.g., injecting a second volume of an injectable composition into the eye of the subject). For example, the composition may be administered to the subject once a week, once every two weeks, once every three weeks, once every four weeks, once a month, once every two months, or once every three months.

### D. Subjects

The subject may be selected from rodents, lagomorphs, ovines, porcines, canines, felines, equines, bovines, and primates. In preferred embodiments, the subject is a human or rabbit. The subject may have an eye disease.

In some embodiments, the subject has macular degeneration, such as age-related macular degeneration ("AMD") or dry macular degeneration, or retinal vein occlusion ("RVO"). In some embodiments, the subject has dry AMD. The subject may have geographic atrophy, such as geographic atrophy that impairs the vision of the subject. The subject may be at risk for developing geographic atrophy. The composition may be administered to prevent geographic atrophy. The subject may have vision loss or the subject may be at risk for developing vision loss, *e.g.,* due to macular degeneration. The subject may be at risk for developing wet AMD The composition may be administered to prevent the development of wet AMD

In some embodiments, the subject has retinal vein occlusion ("RVO"), e.g., central retinal vein occlusion ("CRVO") or branch retinal vein occlusion ("BRVO"). The subject may have ischemic retinal vein occlusion or non-ischemic retinal vein occlusion. The subject may have vision loss or the subject may be at risk for developing vision loss, *e.g.,* due to retinal vein occlusion. The subject may have glaucoma or the subject may be at risk for developing glaucoma, e.g., due to retinal vein occlusion. The subject may have macular edema or the subject may be at risk for developing macular edema, *e.g.,* due to retinal vein occlusion.

### EXEMPLIFICATION

### Example 1 - in vitro precipitation of sorafenib into PBS

30 mg of sorafenib free base is dissolved in 10 mL of absolute ethanol. Approximately 200 µL of the sorafenib composition is loaded into a 1 mL syringe through a 1.5 inch, 19-gauge, 5-micron filter needle. The filter needle is replaced with a 30-gauge, 0.5 inch injection needle. Approximately 50 µL of the sorafenib solution is injected into a test tube containing 4 mL of phosphate-buffered saline ("PBS"), and the solution is mixed by vortexing. The test tube is centrifuged at low speed to pellet the precipitate. The supernatant is removed, and the sorafenib concentration is determined by HPLC. 4 mL of fresh PBS is added to the precipitate and the solution is gently vortexed. After 24 hours, the test tube is centrifuged at low speed to pellet the precipitate. The supernatant is removed and the sorafenib concentration is determined by HPLC.

### Example 2 - administration of sorafenib to the eye of a rabbit

A 3 mg/mL solution of sorafenib in ethanol is prepared as in example 1. Approximately 200 µL of the sorafenib composition is loaded into a 1 mL syringe through a 1.5 inch, 19-gauge, 5-micron filter needle. The filter needle is replaced with a 30-gauge, 0.5 inch injection needle. Approximately 50 µL of the sorafenib solution is injected into the vitreous humour of the eye of a rabbit. After 24 hours, a sample of the vitreous humour is drawn and the sorafenib concentration of the sample is determined by HPLC. Another sample is taken after 7 days, and the sorafenib concentration is determined by HPLC.

## Claims

1. A sustained release injectable composition, comprising a poorly water-soluble kinase inhibitor dissolved in a water-miscible organic solvent, wherein:
the concentration of the kinase inhibitor in the composition is at least 200 µg/mL; and
the concentration of the kinase inhibitor in the composition is at least 100 times higher than the solubility of the kinase inhibitor in water at 37°C.

2. The sustained release injectable composition of claim 1, wherein the concentration of the kinase inhibitor in the composition is at least 800 µg/mL.

3. The sustained release injectable composition of claim 1 or 2, wherein the concentration of the kinase inhibitor in the composition is at least 200 times higher than the solubility of the kinase inhibitor in water at 37°C.

4. The sustained release injectable composition of any one of the preceding claims, wherein the solubility of the kinase inhibitor in water is less than 10 µg/mL.

5. The sustained release injectable composition of any one of the preceding claims, wherein the organic solvent is ethanol.

6. The sustained release injectable composition of any one of the preceding claims, wherein the kinase inhibitor is selected from bafetinib, bosutinib, dasatinib, imatinib, nilotinib, ponatinib, radotinib, SU6656, apatinib, axitinib, cabozantinib, cediranib, crenolanib, foretinib, lenvatinib, linifanib, masitinib, motesanib, nintedanib, pazopanib, pegaptanib, regorafenib, semaxanib, sorafenib, sunitinib, tivozanib, toceranib, vandetanib, and vatalanib;
optionally wherein the kinase inhibitor is sorafenib present in a concentration in the range of 800 µg/mL to 4000 µg/mL.

7. The sustained release injectable composition of any one of the preceding claims, wherein the composition is a liquid.

8. The sustained release injectable composition according to any one of the preceding claims, wherein the injectable composition is in a vial or a syringe.

9. The sustained release injectable composition according to claim 8, wherein
(a) the vial comprises 50 µL to 500 µL of the injectable composition; and/or
(b) the vial comprises 50 µg to 1000 µg of sorafenib; and/or
(c) the vial is glass.

10. The sustained release injectable composition according to claim 8, wherein the syringe
(a) comprises 50 µL to 500 µL of the injectable composition; and/or
(b) comprises 50 µg to 1000 µg of sorafenib; and/or
(c) has a volume of 50 µL to 1 mL; and/or
(d) comprises glass, polypropylene, or polytetrafluoroethylene; and/or
(e) comprises a filter; and/or
(f) comprises a needle.

11. The sustained release injectable composition according to claim 10, wherein the filter
(a) comprises pores with a pore size of 0.2 µm to 5 µm; and/or
(b) is a filter needle.

12. The sustained release injectable composition according to claim 10, wherein the needle
(a) comprises metal, such as stainless steel; and/or
(b) is 6.35 mm (0.25 inches) long to 25.4 mm (1.0 inches) long, optionally 12.7 mm (0.5 inches) long; and/or
(c) is 28 gauge to 33 gauge, optionally 29 gauge, 30 gauge, 31 gauge, or 32 gauge.

13. A kit comprising the vial according to claim 8 or 9 and a syringe.

14. A sustained release injectable composition according to claims 1-12 for use in preventing or treating an eye disease in a subject, optionally wherein,
the composition is for injecting into the vitreous of the eye and comprises 10 µL to 100 µL, or 50 µL, of the injectable composition; and/or
the injectable composition is filtered prior to injection into the eye, optionally, by drawing the composition through a filter needle; and/or
wherein the subject is selected from rodents, lagomorphs, ovines, porcines, canines, felines, equines, bovines, and primates, optionally a human; and/or
wherein the subject is at risk of developing wet age-related macular degeneration; and/or
wherein the subject is at risk of geographic atrophy; and/or
wherein the subject is at risk of developing vision loss; and/or
wherein the eye disease in the subject is age-related macular degeneration; and/or
wherein the eye disease in the subject is dry macular degeneration; and/or
wherein the eye disease in the subject is dry age-related macular degeneration; and/or
wherein the eye disease in the subject is wet macular degeneration; and/or
wherein the eye disease in the subject is wet age-related macular degeneration; and/or
wherein the eye disease in the subject is geographic atrophy; and/or
wherein the eye disease in the subject is vision loss; and/or
wherein the eye disease in the subject is retinal vein occlusion, optionally
wherein the subject has non-ischemic retinal vein occlusion or ischemic retinal vein occlusion.

## Patentansprüche

1. Injizierbare Zusammensetzung mit anhaltender Freisetzung, die einen schlecht wasserlöslichen Kinaseinhibitor umfasst, der in einem wassermischbaren organischen Lösungsmittel gelöst ist, wobei:
die Konzentration des Kinaseinhibitors in der Zusammensetzung mindestens 200 µg/ml beträgt; und
die Konzentration des Kinaseinhibitors in der Zusammensetzung mindestens 100-mal höher ist als die Löslichkeit des Kinaseinhibitors in Wasser bei 37 °C.

2. Injizierbare Zusammensetzung mit anhaltender Freisetzung nach Anspruch 1, wobei die Konzentration des Kinaseinhibitors in der Zusammensetzung mindestens 800 µg/ml beträgt.

3. Injizierbare Zusammensetzung mit anhaltender Freisetzung nach Anspruch 1 oder 2, wobei die Konzentration des Kinaseinhibitors in der Zusammensetzung mindestens 200-mal höher ist als die Löslichkeit des Kinaseinhibitors in Wasser bei 37 °C.

4. Injizierbare Zusammensetzung mit anhaltender Freisetzung nach einem der vorstehenden Ansprüche, wobei die Löslichkeit des Kinaseinhibitors in Wasser weniger als 10 µg/ml beträgt.

5. Injizierbare Zusammensetzung mit anhaltender Freisetzung nach einem der vorstehenden Ansprüche, wobei das organische Lösungsmittel Ethanol ist.

6. Injizierbare Zusammensetzung mit anhaltender Freisetzung nach einem der vorstehenden Ansprüche, wobei der Kinaseinhibitor aus Folgenden ausgewählt ist:
Bafetinib, Bosutinib, Dasatinib, Imatinib, Nilotinib, Ponatinib, Radotinib, SU6656, Apatinib, Axitinib, Cabozantinib, Cediranib, Crenolanib, Foretinib, Lenvatinib, Linifanib, Masitinib, Motesanib, Nintedanib, Pazopanib, Pegaptanib, Regorafenib, Semaxanib, Sorafenib, Sunitinib, Tivozanib, Toceranib, Vandetanib und Vatalanib;
optional wobei der Kinaseinhibitor Sorafenib ist, das in einer Konzentration in dem Bereich von 800 µg/ml bis 4000 µg/ml vorliegt.

7. Injizierbare Zusammensetzung mit anhaltender Freisetzung nach einem der vorstehenden Ansprüche, wobei die Zusammensetzung eine Flüssigkeit ist.

8. Injizierbare Zusammensetzung mit anhaltender Freisetzung nach einem der vorstehenden Ansprüche, wobei die injizierbare Zusammensetzung in einem Fläschchen oder einer Spritze vorliegt.

9. Injizierbare Zusammensetzung mit anhaltender Freisetzung nach Anspruch 8, wobei
(a) das Fläschchen 50 µl bis 500 µl der injizierbaren Zusammensetzung umfasst; und/oder
(b) das Fläschchen 50 µg bis 1000 µg Sorafenib umfasst; und/oder
(c) das Fläschchen Glas ist.

10. Injizierbare Zusammensetzung mit anhaltender Freisetzung nach Anspruch 8, wobei die Spritze
(a) 50 µl bis 500 µl der injizierbaren Zusammensetzung umfasst; und/oder
(b) 50 µg bis 1000 µg Sorafenib umfasst; und/oder
(c) ein Volumen von 50 µl bis 1 ml aufweist; und/oder
(d) Glas, Polypropylen oder Polytetrafluorethylen umfasst; und/oder
(e) einen Filter umfasst; und/oder
(f) eine Nadel umfasst.

11. Injizierbare Zusammensetzung mit anhaltender Freisetzung nach Anspruch 10, wobei der Filter
(a) Poren mit einer Porengröße von 0,2 µm bis 5 µm umfasst; und/oder
(b) eine Filternadel ist.

12. Injizierbare Zusammensetzung mit anhaltender Freisetzung nach Anspruch 10, wobei die Nadel
(a) Metall, wie zum Beispiel Edelstahl, umfasst; und/oder
(b) 6,35 mm (0,25 Zoll) lang bis 25,4 mm (1,0 Zoll) lang, optional 12,7 mm (0,5 Zoll) lang ist; und/oder
(c) 28 Gauge bis 33 Gauge, optional 29 Gauge, 30 Gauge, 31 Gauge oder 32 Gauge aufweist.

13. Kit, der das Fläschchen nach Anspruch 8 oder 9 und eine Spritze umfasst.

14. Injizierbare Zusammensetzung mit anhaltender Freisetzung nach den Ansprüchen 1-12 für die Verwendung bei der Prävention oder Behandlung einer Augenerkrankung bei einem Subjekt, optional wobei
die Zusammensetzung für die Injektion in den Glaskörper des Auges vorgesehen ist und 10 µl bis 100 µl oder 50 µl der injizierbaren Zusammensetzung umfasst; und/oder
die injizierbare Zusammensetzung vor der Injektion in das Auge, optional durch Ziehen der Zusammensetzung durch eine Filternadel, gefiltert wird; und/oder
wobei das Subjekt aus Folgenden ausgewählt ist: Nagetieren, Hasentieren, Schafen, Schweinen, Hunden, Katzen, Einhufern, Rindern und Primaten, optional einem Menschen; und/oder
wobei bei dem Subjekt das Risiko der Entwicklung von feuchter altersbedingter Makuladegeneration besteht; und/oder
wobei bei dem Subjekt das Risiko von geographischer Atrophie besteht; und/oder
wobei bei dem Subjekt das Risiko der Entwicklung von Sehverlust besteht; und/oder
wobei die Augenerkrankung bei dem Subjekt altersbedingte Makuladegeneration ist; und/oder
wobei die Augenerkrankung bei dem Subjekt trockene Makuladegeneration ist; und/oder
wobei die Augenerkrankung bei dem Subjekt trockene altersbedingte Makuladegeneration ist; und/oder
wobei die Augenerkrankung bei dem Subjekt feuchte Makuladegeneration ist; und/oder
wobei die Augenerkrankung bei dem Subjekt feuchte altersbedingte Makuladegeneration ist; und/oder
wobei die Augenerkrankung bei dem Subjekt geographische Atrophie ist; und/oder
wobei die Augenerkrankung bei dem Subjekt Sehverlust ist; und/oder
wobei die Augenerkrankung bei dem Subjekt retinaler Venenverschluss ist, optional
wobei das Subjekt nicht-ischämischen retinalen Venenverschluss oder ischämischen retinalen Venenverschluss hat.

## Revendications

1. Composition injectable à libération prolongée, comprenant un inhibiteur de kinase peu hydrosoluble dissous dans un solvant organique miscible avec l'eau, dans laquelle :
la concentration en inhibiteur de kinase dans la composition est d'au moins 200 µg/ml ; et
la concentration en inhibiteur de kinase dans la composition est au moins 100 fois supérieure à la solubilité de l'inhibiteur de kinase dans l'eau à 37°C.

2. Composition injectable à libération prolongée selon la revendication 1, dans laquelle la concentration en inhibiteur de kinase dans la composition est d'au moins 800 µg/ml.

3. Composition injectable à libération prolongée selon la revendication 1 ou 2, dans laquelle la concentration en inhibiteur de kinase dans la composition est au moins 200 fois supérieure à la solubilité de l'inhibiteur de kinase dans l'eau à 37°C.

4. Composition injectable à libération prolongée selon l'une quelconque des revendications précédentes, dans laquelle la solubilité de l'inhibiteur de kinase dans l'eau est inférieure à 10 µg/ml.

5. Composition injectable à libération prolongée selon l'une quelconque des revendications précédentes, dans laquelle le solvant organique est l'éthanol.

6. Composition injectable à libération prolongée selon l'une quelconque des revendications précédentes, dans laquelle l'inhibiteur de kinase est choisi parmi le bafétinib, le bosutinib, le dasatinib, l'imatinib, le nilotinib, le ponatinib, le radotinib, SU6656, l'apatinib, l'axitinib, le cabozantinib, le cédiranib, le crénolanib, le forétinib, le lenvatinib, le linifanib, le masitinib, le motésanib, le nintédanib, le pazopanib, le pégaptanib, le régorafénib, le sémaxanib, le sorafénib, le sunitinib, le tivozanib, le tocéranib, le vandétanib, et le vatalanib ;
éventuellement dans laquelle l'inhibiteur de kinase est le sorafénib présent selon une concentration dans la plage allant de 800 µg/ml à 4000 µg/ml.

7. Composition injectable à libération prolongée selon l'une quelconque des revendications précédentes, la composition étant un liquide.

8. Composition injectable à libération prolongée selon l'une quelconque des revendications précédentes, la composition injectable se trouvant dans un flacon ou une seringue.

9. Composition injectable à libération prolongée selon la revendication 8, dans laquelle
(a) le flacon comprend de 50 µl à 500 µl de la composition injectable ; et/ou
(b) le flacon comprend de 50 µg à 1000 µg de sorafénib ; et/ou
(c) le flacon est constitué de verre.

10. Composition injectable à libération prolongée selon la revendication 8, dans laquelle la seringue
(a) comprend de 50 µl à 500 µl de la composition injectable ; et/ou
(b) comprend de 50 µg à 1000 µg de sorafénib ; et/ou
(c) possède un volume allant de 50 µl à 1 ml ; et/ou
(d) comprend du verre, du polypropylène, ou du polytétrafluoroéthylène ; et/ou
(e) comprend un filtre ; et/ou
(f) comprend une aiguille.

11. Composition injectable à libération prolongée selon la revendication 10, dans laquelle le filtre
(a) comprend des pores ayant une taille de pores allant de 0,2 µm à 5 µm ; et/ou
(b) est une aiguille à filtre.

12. Composition injectable à libération prolongée selon la revendication 10, dans laquelle l'aiguille
(a) comprend du métal, tel que de l'acier inoxydable ; et/ou
(b) est d'une longueur allant de 6,35 mm (0,25 pouce) à 25,4 mm (1,0 pouce), éventuellement d'une longueur de 12,7 mm (0,5 pouce) ; et/ou
(c) va de 28 gauge à 33 gauge, éventuellement est de 29 gauge, 30 gauge, 31 gauge, ou 32 gauge.

13. Kit comprenant le flacon selon la revendication 8 ou 9, et une seringue.

14. Composition injectable à libération prolongée selon les revendications 1-12, pour une utilisation destinée à la prévention ou au traitement d'une maladie oculaire chez un sujet, éventuellement
la composition étant destinée à une injection dans le corps vitré de l'œil et comprenant de 10 µl à 100 µl, ou 50 µl, de la composition injectable ; et/ou
la composition injectable étant filtrée préalablement à l'injection dans l'œil, éventuellement par le passage de la composition dans une aiguille à filtre ; et/ou
le sujet étant choisi parmi les rongeurs, les lagomorphes, les ovins, les porcins, les canidés, les félidés, les équidés, les bovins, et les primates, éventuellement un être humain ; et/ou
le sujet risquant de développer une dégénérescence maculaire exsudative liée à l'âge ; et/ou
le sujet risquant de développer une atrophie géographique ; et/ou
le sujet risquant de développer une perte de vision ; et/ou
la maladie oculaire chez le sujet étant la dégénérescence maculaire liée à l'âge ; et/ou
la maladie oculaire chez le sujet étant la dégénérescence maculaire sèche ; et/ou
la maladie oculaire chez le sujet étant la dégénérescence maculaire sèche liée à l'âge ; et/ou
la maladie oculaire chez le sujet étant la dégénérescence maculaire exsudative ; et/ou
la maladie oculaire chez le sujet étant la dégénérescence maculaire exsudative liée à l'âge ; et/ou
la maladie oculaire chez le sujet étant l'atrophie géographique ; et/ou
la maladie oculaire chez le sujet étant la perte de vision ; et/ou
la maladie oculaire chez le sujet étant l'occlusion veineuse rétinienne ; éventuellement
le sujet souffrant d'une occlusion veineuse rétinienne non ischémique ou d'une occlusion veineuse rétinienne ischémique.
